# EUROPÄISCHE PATENTANMELDUNG

(11) **EP 4 778 481 A1**
(43) Veröffentlichungstag der Anmeldung: **22.07.2026**
(21) Anmeldenummer: 26152543.0
(22) Anmeldetag: 19.01.2026
(51) Int. Cl.: A61B 18/14, A61B 18/12

(54) **GESPÜLTER BALLONKATHETER ZUR ABLATION VON GEWEBE**

(30) Priorität: 20.01.2025 DE 102025101828
(71) Anmelder: Stockert GmbH, 79111 Freiburg im Breisgau (DE)
(72) Erfinder: REHBERGER, Frank, 79111 Freiburg im Breisgau (DE); STORK, David, 79114 Freiburg (DE); CORCORAN, Colin, 79227 Schallstadt (DE)
(74) Vertreter: Frenkel, Matthias Alexander

(57) **Zusammenfassung**

Die Erfindung betrifft allgemein eine Applikationsvorrichtung, insbesondere eine Kathetervorrichtung, ein System mit einer solchen Vorrichtung zur Ablation von Gewebe und ein Verfahren zur Ablation von Gewebe.

## Beschreibung

Die Erfindung betrifft allgemein eine Applikationsvorrichtung, insbesondere eine Kathetervorrichtung, ein System mit einer solchen Vorrichtung zur Ablation von Gewebe und ein Verfahren zur Ablation von Gewebe.

Die Behandlung von Gewebe mittels elektrischer Energie, wie beispielsweise Hochfrequenz (HF) -Strömen oder gepulsten elektrischen Feldern, ist eine etablierte klinische Technik. Bei der HF-Ablation wird ein Applikator in das Gewebe eingebracht und ein Hochfrequenzstrom erzeugt. Bei der gepulsten Feldablation (engl. Pulsed Field Ablation (PFA)) werden kurze elektrische Hochspannungs-Pulse und damit verbundene hohe elektrische Feldstärken, die auf das Gewebe einwirken, ausgenutzt. Diese Anwendungsmethode wird als nicht-thermisches Verfahren kategorisiert, da sie auf der Abgabe kurzer Pulse mit hoher Spannungsamplitude basiert, die ein lokal starkes elektrisches Feld im Bereich von bis zu mehreren Tausend Volt pro Zentimeter zwischen aktiven Elektrodenpaaren erzeugen. Diese Feldstärke führt zur kurzzeitigen Bildung von Poren in den Zellmembranen. Überschreitet das elektrische Feld einen bestimmten Schwellenwert, der für die Bildung von Poren in den Lipiddoppelschichten der Zellmembranen erforderlich ist, und ist das Gewebe diesem Feld über einen kritischen Zeitraum ausgesetzt, sterben die Zellen durch Apoptose (eine Form des programmierten Zelltods).

Zur Behandlung von Herzrhythmusstörungen, wie z. B. Vorhofflimmern, werden hierbei Applikatoren verwendet, welche oftmals Katheter mit Elektroden auf sog. Körbchen-Strukturen, Ballons oder auf einen Schlauch montierten Ringelektroden sind.

Die vorliegende Erfindung ordnet sich im Bereich der minimal-invasiven medizinischen Verfahren ein, mit einem speziellen Fokus auf der Ablationstechnik. Insbesondere konzentriert sie sich auf die elektrische Energieabgabe via HF-Ablation und irreversibler Elektroporation (IRE), der sogenannten gepulsten Feldablation (engl. Pulsed Field Ablation (PFA)) von Gewebe. Die IRE ist eine fortschrittliche Methode, die in der interventionellen Medizin zur gezielten Behandlung von Gewebe eingesetzt wird. Diese Technik stellt einen bedeutenden Fortschritt in der medizinischen Therapie dar und wird bereits gezielt für die Ablation von Herzgewebe eingesetzt, unter besonderer Berücksichtigung der Gewebeselektivität, reduzierter Behandlungszeiten und der Minimierung von Risiken konventioneller Behandlungsmethoden.

Aus dem Stand der Technik ist die US 11690671 B1 bekannt, die einen Gewebe-Ablationskatheter mit einem Isolator zwischen innerer und äußerer Elektrode betrifft. Die Anordnung offenbart an einem distalen Ende versetzte Elektroden. Ein Material zwischen innerer und äußerer Elektrode ist ein Isolator. Diese Katheterkonfiguration erzeugt ein elektrisches Feld, welches sich um die Spitze des Katheters wölbt.

Es besteht ein Bedarf an einer verbesserten Applikationsvorrichtung, insbesondere Kathetervorrichtung, sowie an einem zugehörigen System und Verfahren zur Ablation von Gewebe.

Hierfür werden eine Applikationsvorrichtung, insbesondere eine Kathetervorrichtung, nach Anspruch 1, ein System nach Anspruch 12 und ein Verfahren nach Anspruch 18 vorgeschlagen.

Gemäß einem ersten Aspekt wird eine Applikationsvorrichtung vorgeschlagen. Die Applikationsvorrichtung kann insbesondere als eine Kathetervorrichtung ausgebildet sein. Die Applikationsvorrichtung, insbesondere Kathetervorrichtung, wird hierin oftmals nur kurz als Vorrichtung bezeichnet. Die Vorrichtung kann insbesondere zur Verwendung in der Chirurgie dienen.

Die (Applikations-)Vorrichtung weist einen Schaft auf, der dazu eingerichtet ist, zumindest ein elektrisch leitfähiges Fluid aufzunehmen, insbesondere von einem proximalen Ende des Schafts zu einem distalen Ende des Schafts zu leiten. Die Vorrichtung weist zumindest eine erste Elektrode auf, die auf einem ersten Abschnitt des Schafts angeordnet ist. Die Vorrichtung weist zumindest eine erste Gegenelektrode auf. Die Vorrichtung weist eine erste elektrische Leitung auf, die mit der zumindest einen ersten Elektrode und der zumindest einen ersten Gegenelektrode verbunden ist. Die erste elektrische Leitung ist dazu eingerichtet, ein zu empfangendes elektrisches Signal an die zumindest eine erste Elektrode und die zumindest eine erste Gegenelektrode weiterzuleiten. Die Vorrichtung weist zumindest eine erste, insbesondere elektrisch isolierende, Membran auf. Die zumindest eine erste Membran ist dazu eingerichtet, den ersten Abschnitt des Schafts zu umschließen, mit dem Schaft fluiddicht, insbesondere flüssigkeitsdicht, abzuschließen, und zwischen der zumindest einen ersten Membran und dem ersten Abschnitt des Schafts einen ersten Innenraum zu bilden. Der erste Abschnitt des Schafts ist dazu eingerichtet, das zumindest eine elektrisch leitfähige Fluid in den Innenraum zu leiten. Eine erste Wandung der zumindest einen ersten Membran ist dazu eingerichtet, zumindest teilweise fluiddurchlässig, insbesondere flüssigkeitsdurchlässig, zu sein.

Ein Vorteil der Vorrichtung ist, dass eine elektrische Verbindung zu dem Fluid bewirkt wird, sodass die zumindest teilweise fluiddurchchlässige Membran einen auf einer/der (z.B. zumindest einen ersten) Elektrode abgegebenen Spannungspuls bündelt und sozusagen "virtuelle Elektroden" an Orten erzeugt, an denen das elektrisch leitfähige Fluid aus der Membran austritt. Gleichzeitig wird durch das austretende Fluid der thermische Effekt am Gewebe gezielt reduziert. Gleichzeitig wird die Komplexität verringert, da nur eine elektrische Verbindung benötigt wird.

Dies hat den Vorteil, dass einerseits thermische Effekte durch elektrisch leitfähiges Fluid, bspw. eine Spüllösung, verringert werden können und gleichzeitig kann durch die Membran der Gewebekontakt und eine damit verbundene Okklusion sichergestellt werden.

Das elektrisch leitfähige Fluid kann ein erstes und/oder ein zweites elektrisch leitfähiges Fluid aufweisen oder als ein erstes und/oder ein zweites elektrisch leitfähiges Fluid ausgebildet sein. Der Schaft der Vorrichtung kann dazu eingerichtet sein, das erste und/oder das zweite elektrisch leitfähige Fluid aufzunehmen. Der erste Abschnitt des Schafts kann dazu eingerichtet sein, das erste und/oder das zweite elektrisch leitfähige Fluid in den Innenraum zu leiten.

Die zumindest eine erste Membran, insbesondere die erste Wandung, kann zumindest eine erste Perforation aufweisen, aus der das, insbesondere das erste und/oder zweite, elektrisch leitfähige Fluid austreten kann.

Der erste Abschnitt kann zwischen dem distalen Ende und dem proximalen Ende angeordnet sein.

Die Vorrichtung kann zumindest eine zweite Elektrode aufweisen. Die zumindest eine erste und/oder zweite Elektrode kann/können auf einem zweiten und/oder dem ersten Abschnitt des Schafts angeordnet und mit der ersten elektrischen Leitung verbunden sein.

Der zweite Abschnitt kann zwischen dem ersten Abschnitt und dem proximalen Ende angeordnet sein.

Die Vorrichtung kann zumindest eine zweite, insbesondere elektrisch isolierende, Membran aufweisen. Die zumindest eine zweite Membran kann dazu eingerichtet sein, den zweiten Abschnitt des Schafts zu umschließen, mit dem Schaft fluiddicht, insbesondere flüssigkeitsdicht, abzuschließen, und zwischen der zumindest einen zweiten Membran und dem zweiten Abschnitt des Schafts einen zweiten Innenraum zu bilden.

Der zweite Abschnitt des Schafts kann dazu eingerichtet sein, ein/das, insbesondere das erste und/oder zweite, elektrisch leitfähige Fluid in den zweiten Innenraum zu leiten. Eine zweite Wandung der zumindest einen zweiten Membran kann dazu eingerichtet sein, fluiddurchlässig, insbesondere flüssigkeitsdurchlässig, zu sein.

Die zumindest eine zweite Membran, insbesondere die zweite Wandung, kann zumindest eine zweite Perforation aufweisen, aus der das, insbesondere das erste und/oder zweite, elektrisch leitfähige Fluid austreten kann.

Der erste und/oder der zweite Innenraum kann/können zumindest eine erste Fluidkammer und/oder eine zweite Fluidkammer aufweisen. Die zumindest eine erste und/oder zweite Fluidkammer kann/können dazu eingerichtet sein, den ersten und/oder zweiten Abschnitt des Schafts fluiddicht zu umschließen/mit dem ersten und/oder zweiten Abschnitt des Schafts fluiddicht verbunden zu sein. Die zumindest eine erste Fluidkammer kann dazu eingerichtet sein, das oder ein erstes elektrisch leitfähiges Fluid zu zumindest einer ersten Teilfläche einer Innenseitenfläche der ersten und/oder zweiten Membran zu leiten. Die zumindest eine zweite Fluidkammer kann dazu eingerichtet sein, das oder ein zweites elektrisch leitfähiges Fluid zu zumindest einer zweiten Teilfläche der Innenseitenfläche der ersten und/oder zweiten Membran zu leiten. Die erste und/oder zweite Fluidkammer kann/können die zumindest eine erste und/oder die zumindest eine zweite Elektrode einschließen. Die zumindest eine erste und/oder zweite Elektrode kann auf einem ersten und/oder zweiten Teil des ersten oder des zweiten Abschnitts des Schafts angeordnet sein.

Die zumindest eine erste Fluidkammer kann mit der zumindest einen ersten Teilfläche einer Innenseitenfläche der zumindest einen ersten und/oder der zumindest einen zweiten Membran fluiddicht verbunden sein. Die zumindest eine zweite Fluidkammer kann mit der zumindest einen zweiten Teilfläche der Innenseitenfläche der zumindest einen ersten und/oder der zumindest einen zweiten Membran fluiddicht verbunden sein.

Die zumindest eine erste und/oder zweite Fluidkammer kann/können einen beliebigen Winkelbereich (um eine Längsachse der ersten und/oder zweiten Membran) zwischen 45° und 360° einnehmen/überstreifen. Die zumindest eine erste und/oder zweite Fluidkammer kann/können jeweils einen beliebigen Winkelbereich einnehmen/überstreifen, sodass sich ein Gesamtwinkelbereich beider Fluidkammern zwischen 45° und 360° ergibt (z. B. erster Winkelbereich 90°, zweiter Winkelbereich 180°, Gesamtwinkelbereich 270°).

Die Ausbildung einer oder mehrerer Fluidkammern hat den Vorteil, dass nur bestimmte Stellen des Gewebes mittels der Energie appliziert werden. Damit wird ein unnötiger Energieeintrag in nicht zu abladierendes Gewebe verhindert.

Die zumindest eine erste, die zumindest eine zweite Elektrode, die zumindest eine erste, zweite, dritte (siehe unten) Gegenelektrode und/oder die Membran(en), insbesondere die zumindest eine erste und/oder zweite Perforation, kann/können ein bioresorbierbares, insbesondere in Köperflüssigkeiten lösliches, Material aufweisen oder mit einem bioresorbierbaren, insbesondere einem in Köperflüssigkeiten löslichen, Material oder einer entsprechenden Materialschicht bedeckt sein.

Die zumindest eine erste Membran kann zwischen dem distalen Ende (des Schafts) und dem proximalen Ende (des Schafts) angeordnet sein. Die zumindest eine zweite Membran kann zwischen der zumindest einen ersten Membran und dem proximalen Ende des Schafts angeordnet sein. Die zumindest eine erste und/oder die zumindest eine zweite Membran kann/können als Ballon ausgebildet sein.

Die Vorrichtung kann ferner zumindest eine Begrenzungsstruktur aufweisen. Die zumindest eine Begrenzungsstruktur kann einen zweiten Teil des Schafts fluiddicht, insbesondere flüssigkeitsdicht, umschließen. Die zumindest eine Begrenzungsstruktur kann dazu eingerichtet sein, das aus der ersten Wandung, insbesondere aus der zumindest einen ersten Perforation, austretende elektrische leitfähige Fluid in einer Ausbreitungsrichtung zu begrenzen oder zu hemmen und/oder entlang des Schafts verschiebbar zu sein oder verschoben zu werden. Die zumindest eine Begrenzungsstruktur kann zwischen der zumindest einen ersten Membran und dem proximalen Ende angeordnet sein. Die zumindest eine Begrenzungsstruktur kann dazu eingerichtet sein, fluiddicht mit dem Schaft abzuschließen.

Die Vorrichtung kann eine Ablauföffnung/Drainageöffnung aufweisen. Die Ablauföffnung kann auf dem Schaft angeordnet sein oder in den Schaft integriert sein. Die Ablauföffnung kann auf dem Schaft zwischen der zumindest einen ersten Membran und der zumindest einen Begrenzungsstruktur oder zwischen der zumindest einen ersten Membran und der zumindest einen zweiten Membran angeordnet sein. Die Ablauföffnung kann dazu eingerichtet sein, das aus der ersten Wandung, insbesondere aus der zumindest einen ersten Perforation, und/oder aus der zweiten Wandung, insbesondere aus der zumindest einen zweiten Perforation, ausgetretene elektrisch leitfähige Fluid, z. B. in den Schaft, abzuführen. Der Schaft kann dazu eingerichtet sein, das abgeführte elektrisch leitfähige Fluid, z. B. zu dem proximalen Ende oder über das proximale Ende, aus dem Schaft zu leiten. Der Schaft kann eine in dem Schaft angeordnete und mit der Ablauföffnung verbundenen Ablaufleitung aufweisen.

Es kann/können zumindest eine erste, zumindest eine zweite, und/oder eine zumindest eine dritte Gegenelektrode vorgesehen sein. Beispielsweise kann/können zumindest eine erste, zumindest eine zweite und/oder eine zumindest eine dritte Gegenelektrode vorgesehen zwischen: dem distalen Ende und der zumindest einen ersten Membran, oder zwischen der zumindest einen ersten Membran und dem proximalen Ende, oder zwischen der zumindest einen ersten und der zumindest einen zweiten Membran, oder zwischen der zumindest einen zweiten Membran und dem proximalen Ende, oder zwischen der zumindest einen ersten Membran und der zumindest einen Begrenzungsstruktur angeordnet sein. Die zumindest eine erste, zumindest eine zweite, und/oder zumindest eine dritte Gegenelektrode kann/können nebeneinander oder getrennt voneinander angeordnet sein. Die zumindest eine erste und/oder zumindest eine zweite und/oder zumindest eine dritte Gegenelektrode kann/können als Körperelektrode ausgebildet sein.

Die zumindest eine erste und/oder die zumindest eine zweite Perforation kann als zumindest eine Perforationslinie ausgebildet sein. Die zumindest eine Perforationslinie kann entlang einer Längsachse der ersten und/oder zweiten Membran, insbesondere von einem mittleren/mittigen Membranumfang beginnend oder von einem ersten oder zweiten Ende der ersten und/oder zweiten Membran beginnend, in Längsachsrichtung, hin zu einem zweiten oder ersten Ende der ersten und/oder zweiten Membran ausgerichtet sein.

Die zumindest eine erste und/oder die zumindest eine zweite Perforation kann/können als eine Vielzahl von Perforationslinien ausgebildet sein, die jeweils entlang einer Längsachse der ersten und/oder zweiten Membran, insbesondere von einem mittleren/mittigen Umfang beginnend oder von einem ersten oder zweiten Ende der ersten und/oder zweiten Membran beginnend, in Längsachsrichtung, hin zu einem zweiten und/oder ersten Ende der ersten und/oder zweiten Membran, ausgerichtet sein. Die zumindest eine erste und/oder die zumindest eine zweite Perforation kann/können als zumindest eine Perforationslinie oder als eine Vielzahl von Perforationslinien oder als eine Vielzahl von Perforationen ausgebildet sein, die entlang oder quer eines, insbesondere mittleren/mittigen, Umfangs der ersten und/oder zweiten Membran angeordnet oder verteilt sein können.

Die zumindest eine erste und/oder zweite Teilfläche der Innenseite kann (z. B. ausschließlich) mit der zumindest einen ersten und/oder mit der zumindest einen zweiten Perforation korrespondieren. Die zumindest eine erste und/oder zweite Teilfläche der Innenseite kann (z. B. ausschließlich) mit der zumindest einen Perforationslinie korrespondieren. Der zumindest eine Teil der Innenseite kann (z. B. ausschließlich) mit einer oder mehreren Perforationslinie(n) der Vielzahl von Perforationslinien korrespondieren.

Die zumindest eine erste und/oder zweite Teilfläche der Innenseitenfläche kann einem vollständigen Areal der Innenseitenfläche entsprechen, der sich von dem ersten Ende der ersten oder zweiten Membran hin zu, insbesondere entlang der Längsachse der ersten und/oder zweiten Membran, dem mittleren/mittigen Umfang ergibt/erstreckt. Die zumindest eine erste und/oder zweite Teilfläche der Innenseite kann einem Bereich der Innenseitenfläche entsprechen, der sich von dem ersten Ende der ersten oder zweiten Membran beginnend zu dem zweiten Ende der entsprechenden Membran ergibt/erstreckt.

Der erste und/oder der zweite Abschnitt kann eine Befüllungsöffnung aufweisen, wobei über das proximale Ende der erste und/oder der zweite Innenraum mit dem elektrisch leitfähigen Fluid befüllbar ist oder befüllt wird. Der erste und/oder der zweite Abschnitt kann eine Entlüftungsöffnung aufweisen, wobei der erste und/oder der zweite Innenraum über das proximale Ende entlüftbar ist oder entlüftet wird.

Der Schaft kann eine in dem Schaft angeordnete und mit der Entlüftungsöffnung verbundene Entlüftungsleitung aufweisen. Der Schaft kann eine in dem Schaft angeordnete und mit der Befüllungsöffnung verbundene erste und/oder zweite Befüllungsleitung aufweisen.

Die erste und/oder die zweite Membran kann, insbesondere jeweils, ein erstes Ende und/oder ein zweites Ende aufweisen. In einer (außerhalb des Körpers) lotrecht orientierten Vorrichtungslängsachse kann der Innenraum entlüftet werden, wenn der Innenraum mit der elektrische leitfähigen Flüssigkeit befüllt wird. Der Innenraum kann entlüftet werden, wenn die Vorrichtung lotrecht orientiert/gehalten wird und der Innenraum mit einer elektrisch leitfähigen Flüssigkeit befüllt wird.

Die Vorrichtung, der erste Abschnitt und/oder der zweite Abschnitt und/oder eine Außenseite der ersten und/oder zweiten Membran kann/können einen Fluidruckmess-Sensor und/oder einen Fluidflussmess-Sensor aufweisen. Der Fluidruckmess-Sensor und/oder Fluidflussmess-Sensor können jeweils dazu eingerichtet sein, Mess-Daten bereitzustellen. Die Mess-Daten können über eine in dem Schaft vorgesehene oder angeordnete zweite elektrische Leitung ausgelesen werden.

Dies hat den Vorteil, dass eine Druckmessung des Fluids an verschieden Bereichen der Vorrichtung gemessen werden kann, wenn sich jene in einem Lumen eines Patienten befindet, womit eine genauere Aussage über den Grad der Okklusion und die Belastung für den Patienten bestimmt werden kann. Über den Grad der Okklusion kann eine Fluidzufuhr angepasst werden.

Die erste und/oder die zweite elektrische Leitung kann/können von dem elektrisch leitfähigen Fluid elektrisch isoliert sein.

Das proximale Ende des Schafts kann eine Bedieneinheit aufweisen. Die Bedieneinheit kann einen Zu- und Abfluss-Anschluss aufweisen. Der Zu- und Abfluss-Anschluss kann dazu eingerichtet sein, ein zu empfangendes elektrisch leitfähiges Fluid in den Schaft zu leiten, insbesondere den/die Innenraum/Innenräume mit eine/dem zu empfangenden elektrischen leitfähigen Fluid zu befüllen. Der Zu- und Abfluss-Anschluss kann dazu eingerichtet sein, ausgetretenes und abgeführtes elektrisches leitfähiges Fluid aus dem Schaft zu leiten.

Die Bedieneinheit kann einen mit der ersten und/oder der zweiten elektrischen Leitung verbundenen elektrischen Anschluss aufweisen. Der elektrische Anschluss kann dazu eingerichtet sein, zu empfangende elektrische Signale an die erste elektrische Leitung zu senden und/oder elektrische Signale, insbesondere Mess-Daten von dem Fluiddruckmess-Sensor und/oder Fluidflussmess-Sensor, mittels der zweiten elektrischen Leitung zu empfangen und weiterzusenden.

Die Bedieneinheit kann eine, insbesondere als Hämostaseventil ausgebildete, Einführungsöffnung aufweisen. Die Einführungsöffnung kann dazu eingerichtet sein, einen Führungsdraht aufzunehmen. Der Führungsdraht kann zu oder zu und aus einer Austrittöffnung am distalen Ende des Schafts geleitet werden.

Gemäß einem zweiten Aspekt wird ein System zur Ablation von Gewebe vorgeschlagen. Das System weist mindestens eine Vorrichtung gemäß dem ersten Aspekt, mindestens eine mit der Vorrichtung verbundene oder verbindbare Signalgeneratoranordnung, mindestens eine mit der Vorrichtung und/oder Signalgeneratoranordnung verbundene oder verbindbare Steuer- und Auswerteeinheit, und/oder mindestens eine mit der Vorrichtung und/oder Steuer- und Auswerteeinheit verbundene oder verbindbaren Fluidzufuhreinheit auf.

Die Fluidzufuhreinheit kann dazu eingerichtet sein, zumindest ein oder ein erstes und ein zweites elektrisches leitfähiges Fluid bereitzustellen.

Die Steuer- und Auswerteinheit kann dazu eingerichtet sein, einen Volumenstrom der Fluidzufuhreinheit, insbesondere nach einer Einstellung durch einen Nutzer, vorzugeben. Die Fluidzufuhreinheit kann dazu eingerichtet sein, das (oder das erste und/oder zweite) elektrisch leitfähige Fluid entsprechend der Vorgabe bereitzustellen und mittels des Fluiddruckmess-Sensors und/oder des Fluidfluss-Sensors einen Fluiddruck und/oder Fluidfluss zu messen.

Dies hat den Vorteil, dass einerseits thermische Effekte durch ein elektrisch leitfähiges Fluid, bspw. eine Spüllösung, verringert werden und gleichzeitig kann/können durch die Membran(en) der Gewebekontakt und eine damit verbundene Okklusion sichergestellt sowie Staudruck auch quantifiziert werden.

Die Steuer- und Auswerteeinheit kann dazu eingerichtet sein, aus dem gemessenen Fluiddruck und/oder Fluidfluss ein Maß für die Formschlüssigkeit zwischen der/den Membran(en), insbesondere der zumindest einen Perforation, und dem zu abladierenden Gewebe zu bestimmen und auszugeben. Das Maß für die Formschlüssigkeit kann durch eine Abdeckung in Prozent angegeben werden, bspw. 80% Abdeckung. Das Maß für die Formschlüssigkeit kann in Milliliter pro Minute, insbesondere Milliliter pro Minute, einer Rezirkulation, und/oder in Abdeckung pro Prozent angegeben werden.

Das (das erste und/oder das zweite) elektrisch leitfähige Fluid kann als elektrisch leitfähige Flüssigkeit und/oder als elektrisch leitfähiger flüssiger Wirkstoff ausgebildet sein, insbesondere eine vasoaktive Substanz aufweisen oder als solche, insbesondere (flüssiges) Histamin, ausgebildet sein, der bei Kontakt mit einem Gewebe dessen elektrische Leitfähigkeit verändert, insbesondere erhöht oder vermindert. Die vasoaktive Substanz kann vasodilatorisch oder vasostriktiv wirken.

Dies hat den Vorteil, dass der zu abladierende Gewebeabschnitt mit einem Wirkstoff in Kontakt steht, der die elektrische Leitfähigkeit erhöht. Damit wird die Ablation effektiver gestaltet, wobei ein zum abladierenden Gewebeabschnitt benachbarter Gewebeabschnitt, der nicht abladiert werden soll, durch leitfähigkeitsmindernde Substanzen in dessen elektrische Leitfähigkeit vermindert werden und dadurch der Energieeintrag während der Ablation vermindert werden kann.

Das elektrisch leitfähige Fluid, insbesondere die vasoaktive Substanz, kann Bradykinin, Serotonin, Betahistin, Acetylcholin, Dopamin, Noradrenalin, Angiotensin II, Angiotensin II und/oder Katecholamine aufweisen oder als solche oder als Kombination als solcher ausgebildet sein.

Die Signalgeneratoranordnung kann einen ersten Signalgenerator zur Erzeugung eines Radiofrequenz (RF) -Signals und einen zweiten Signalgenerator zur Erzeugung eines Signals für eine gepulste Feldablation aufweisen.

Die Steuer- und Auswerteeinheit kann dazu eingerichtet sein, den ersten Signalgenerator und/oder den zweiten Signalgenerator zur Abgabe eines Signals anzusteuern.

Gemäß einem dritten Aspekt wird ein Verfahren zur Ablation von Gewebe oder zur Abgabe elektrischer Energie an Gewebe, insbesondere Ablation, insbesondere zur irreversiblen Ablation, vorgeschlagen. Das Verfahren umfasst ein Bereitstellen eines Systems. Das System weist eine Applikationsvorrichtung gemäß dem ersten Aspekt auf.

Das System weist eine mit der Applikationsvorrichtung verbundene oder verbindbare Signalgeneratoranordnung auf. Das System weist eine mit der Applikationsvorrichtung und/oder Signalgeneratoranordnung verbundene oder verbindbare Steuer- und Auswerteeinheit auf. Das System weist eine mit der Applikationsvorrichtung und/oder Steuer- und Auswerteeinheit verbundene oder verbindbare Fluidzufuhreinheit auf, die dazu eingerichtet ist, ein elektrisch leitfähiges Fluid, z. B. eine vasoaktive Substanz, bereitzustellen.

Das Verfahren kann ein Einführen der Applikationsvorrichtung in ein Lumen eines Patienten bis zu einem zu abladierenden Gewebeabschnitt umfassen. Das Verfahren kann ein Einstellen der Steuer- und Auswerteeinheit zur Einstellung des Volumenstroms oder ein Einstellen eines Volumenstroms an der Steuer- und Auswerteeinheit umfassen. Das Verfahren umfasst ein Ansteuern der Steuer- und Auswerteeinheit zur Erzeugung eines elektrischen Signals.

Das elektrisch leitfähige Fluid kann eine vasoaktive Substanz aufweisen. Das Verfahren kann ein Feinpositionieren der Applikationsvorrichtung relativ zu dem zu abladierenden Gewebeabschnitt umfassen. Das Verfahren kann ein Abgeben eines elektrischen Signals umfassen. Das Verfahren kann ein Abgeben einer vasoaktiven Substanz umfassen. Das Verfahren kann ein Ausführen der Applikationsvorrichtung aus dem Lumen des Patienten umfassen.

Das Verfahren kann ein Kontaktieren der Applikationsvorrichtung mit dem Gewebe umfassen. Das Verfahren kann ein Applizieren des elektrisch leitfähigen Fluids, z. B. der vasoaktiven Substanz, umfassen. Nach dem Applizieren kann unmittelbar die Ansteuerung der Steuer- und Auswerteeinheit zur Erzeugung eines elektrischen Signals erfolgen.

Die Applikationsvorrichtung kann dazu eingerichtet seom, ein zu empfangendes elektrisches Signal und eine zu empfangende vasoaktive, insbesondere flüssige, Substanz, insbesondere Histamin, an/in das Gewebe weiterzuleiten.

Gemäß einem vierten Aspekt wird ein Verfahren zur Ablation, insbesondere irreversiblen Ablation, von Gewebe oder zur Abgabe elektrischer Energie an Gewebe vorgeschlagen. Das Verfahren umfasst ein Bereitstellen eines Systems. Das System weist eine Vorrichtung gemäß dem ersten Aspekt, eine mit der Vorrichtung verbundene oder verbindbare Signalgeneratoranordnung, eine mit der Vorrichtung und/oder Signalgeneratoranordnung verbundene oder verbindbare Steuer- und Auswerteeinheit und eine mit der Vorrichtung und/oder Steuer- und Auswerteeinheit verbundenen oder verbindbaren Fluidzufuhreinheit auf.

Die Applikationsvorrichtung ist dazu eingerichtet, ein zu empfangendes elektrisches Signal und eine zu empfangende vasoaktive, insbesondere flüssige, Substanz, insbesondere Histamin, an/in das Gewebe weiterzuleiten. Die Fluidzufuhreinheit ist dazu eingerichtet, die vasoaktive Substanz bereitzustellen.

Das Verfahren umfasst ein Kontaktieren der Applikationsvorrichtung mit dem Gewebe. Das Verfahren umfasst ein Applizieren der vasoaktiven Substanz. Das Verfahren umfasst ein Ansteuern der Steuer- und Auswerteeinheit zur Erzeugung eines elektrischen Signals.

Das Verfahren kann ein Einstellen der Steuer- und Auswerteeinheit zur Einstellung des Volumenstroms umfassen. Das Verfahren kann, nach dem Applizieren, ein unmittelbares Ansteuern der Steuer- und Auswerteeinheit zur Erzeugung eines elektrischen Signals umfassen.

Das Verfahren kann ein Einführen der Vorrichtung in ein Lumen eines Patienten bis zu einem zu abladierenden Gewebeabschnitt umfassen. Das Verfahren kann ein Einstellen eines Volumenstroms an der Fluidzufuhreinheit oder Steuer- und Auswerteeinheit umfassen. Das Verfahren kann ein Ansteuern der Steuer- und Auswerteeinheit zur Erzeugung eines elektrischen Signals umfassen.

Weitere Merkmale, Eigenschaften, Vorteile und mögliche Abwandlungen werden für einen Fachmann anhand der nachstehenden Beschreibungen deutlich, in der auf die beigefügten Zeichnungen Bezug genommen ist.

### Kurzbeschreibung der Figuren

Figur 1 zeigt eine schematische Darstellung einer Variante einer Ausführungsform mit einer Membran.
Figur 1a zeigt eine schematische Darstellung einer Variante einer Ausführungsform mit einer Membran und zwei Fluidkammern.
Figur 1b zeigt eine schematische Darstellung einer Variante einer Ausführungsform mit einer Membran und einer Fluidkammer.
Figur 2 zeigt eine schematische Darstellung einer weiteren Variante einer Ausführungsform mit zwei Membranen.
Figur 3 zeigt eine schematische Darstellung einer Variante einer Ausführungsform mit Bedieneinheit.
Figur 4 zeigt eine schematische Darstellung einer Variante einer Ausführungsform mit Drainageöffnungen.
Figur 5 zeigt eine schematische Darstellung einer Variante einer Ausführungsform mit einem Begrenzungselement.
Figur 6 zeigt eine schematische Darstellung einer Variante einer Ausführungsform mit einem Feldlinienverlauf während der Ablation.
Figur 7 zeigt ein Verfahren zur Abgabe elektrischer Energie an Gewebe.
Figur 8 zeigt ein Verfahren zur irreversiblen Ablation.

In den nachstehend beschriebenen Figuren ist die Applikationsvorrichtung beispielhaft jeweils als Kathetervorrichtung ausgebildet.

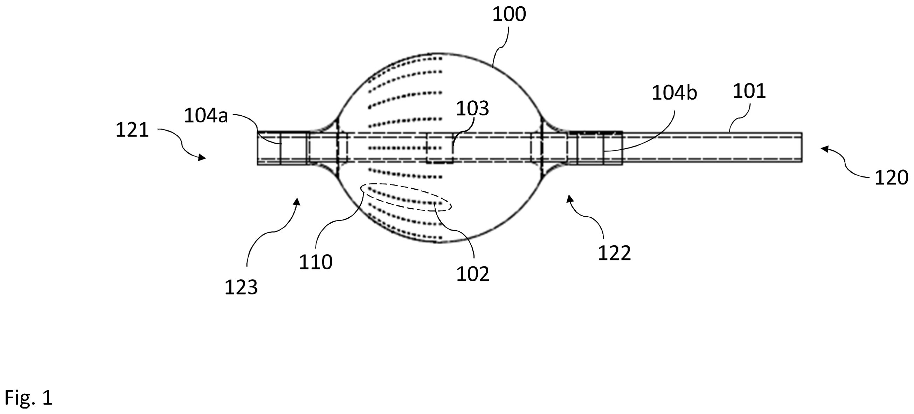

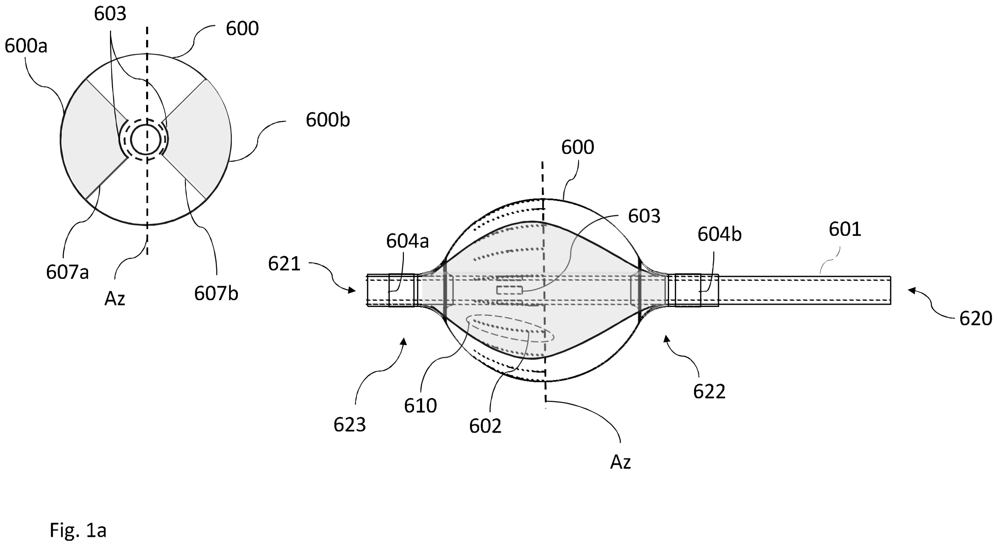

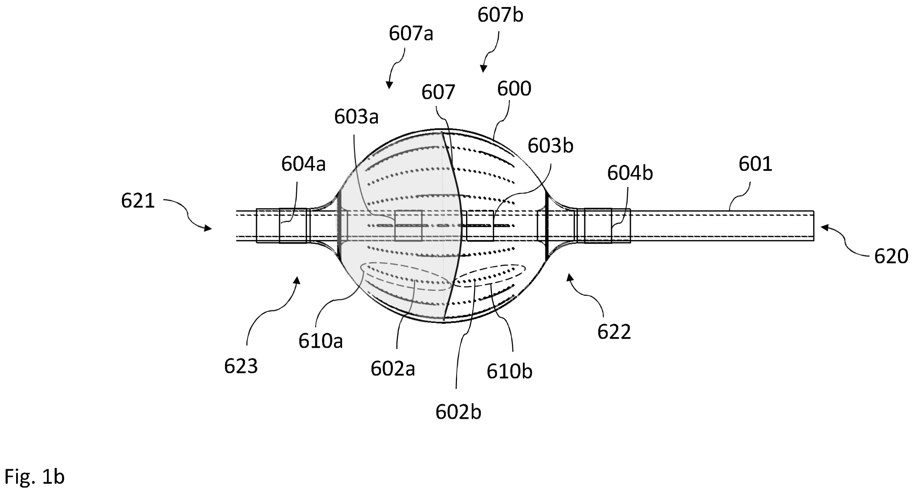

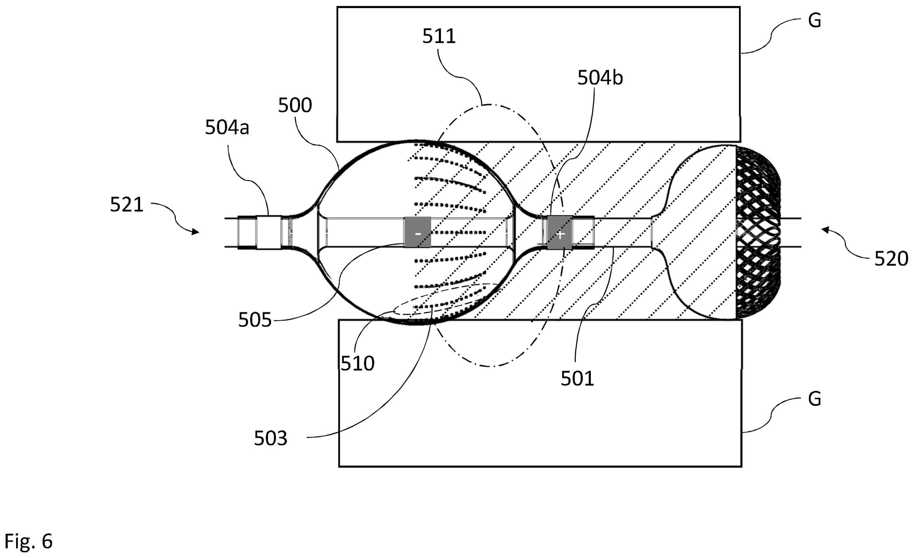

Figur 1 zeigt eine schematische Darstellung einer Variante einer Ausführungsform einer Applikationsvorrichtung, die im gezeigten Beispiel als eine Kathetervorrichtung ausgebildet ist und daher auch als solche bezeichnet wird, mit einer Membran 100. Die abgebildete Variante weist einen Schaft 101 auf, der wiederum ein proximales Ende 120 und ein distales Ende 121 aufweist. Auf einem ersten Abschnitt des Schafts 101 ist eine erste Elektrode 103 angeordnet. Die erste Elektrode 103 wird von einer Membran 100 fluiddicht umschlossen. Anders ausgedrückt sind ein erstes Ende 122 und ein zweites Ende 123 der Membran mit dem Schaft 101 fluiddicht verbunden. Umschließt die Membran 100 den ersten Abschnitt des Schafts 101, bildet sich ein erster Innenraum zwischen der Membran 100 und dem Schaft 101. Die Membran 100 weist zumindest eine einzelne Perforation 102 auf, die in der dargestellten Variante als eine Vielzahl von Perforationslinien ausgebildet ist. Eine der Vielzahl von Perforationslinien ist mit 110 und einer gestrichelten Linienführung markiert. Jede der Vielzahl von Perforationslinien ist entlang einer Längsachse der Membran 100 ausgerichtet. Jede der Perforationslinien beginnt auf dem mittigen Umfang der Membran 100 und endet in der dargestellten Variante an dem zweiten Ende 123 der Membran 100. Neben der ersten Elektrode 103 sind außerhalb des ersten Innenraums auf dem Schaft 101 zwei weitere Gegenelektroden 104a, 104b angeordnet. Das proximale Ende 120 des Schafts 101 ist dazu eingerichtet, ein elektrisch leitfähiges Fluid aufzunehmen und zum distalen Ende 121 des Schafts 101 zu leiten. Der erste Abschnitt des Schafts 101 ist dazu eingerichtet, das elektrische leitfähige Fluid in den ersten Innenraum zu leiten. Der erste Innenraum wird mit dem elektrisch leitfähigen Fluid gefüllt. Das elektrisch leitfähige Fluid tritt aus der Vielzahl von Perforationslinien hinaus. Wird ein elektrisches Signal zwischen der ersten Elektrode 103 und der Gegenelektrode 104a oder 104b angelegt, erfolgt aufgrund des ausgetretenen elektrischen leitfähigen Fluids eine verbesserte Signalführung in das zu abladierende Gewebe, welche in der Figur 6 näher erläutert wird.

Figur 1a zeigt eine schematische Darstellung einer Variante einer Ausführungsform mit einer Membran 600 und zwei Fluidkammern 607a, 607b. Die abgebildete Variante weist einen Schaft 601 auf, der wiederum ein proximales Ende 620 und ein distales Ende 621 aufweist. Auf jeweils einem Teil eines ersten Abschnitts des Schafts 601 ist eine einzelne Elektrode 603 angeordnet. Die Elektroden 603 werden von einer Membran 600 fluiddicht umschlossen. Anders ausgedrückt sind ein erstes Ende 622 und ein zweites Ende 623 der Membran mit dem Schaft 601 fluiddicht verbunden. Umschließt die Membran 600 den ersten Abschnitt des Schafts 601, bildet sich ein erster Innenraum zwischen der Membran 600 und dem Schaft 601. Die Membran 600 weist zumindest eine einzelne Perforation 602 auf, die in der dargestellten Variante als eine Vielzahl von Perforationslinien ausgebildet ist. Eine der Vielzahl von Perforationslinien ist mit 610 und einer gestrichelten Linienführung markiert. Jede der Vielzahl von Perforationslinien ist entlang einer Längsachse der Membran 600 ausgerichtet. Jede der Perforationslinien beginnt auf dem mittigen Umfang der Membran 600 und endet in der dargestellten Variante an dem zweiten Ende 623 der Membran 600. Neben den Elektroden 603 sind außerhalb des ersten Innenraums auf dem Schaft 601 zwei weitere Gegenelektroden 604a, 604b angeordnet. Das proximale Ende 620 des Schafts 601 ist dazu eingerichtet, ein erstes elektrisch leitfähiges Fluid aufzunehmen und zum distalen Ende 621 des Schafts 601 zu leiten.

Der Innenraum weist eine erste und eine zweite Fluidkammer 607a, 607b auf. Jede der Fluidkammern 607a, 607b ist mit jeweils einem Teil des ersten Abschnitts des Schafts 601 fluiddicht verbunden, auf dem eine der Elektroden 603 angeordnet ist. Die erste Fluidkammer 607a ist mit einer ersten Teilfläche 600a einer Innenseitenfläche der Membran 600 fluiddicht verbunden. Die zweite Fluidkammer 607b ist mit einer zweiten Teilfläche 600b der Innenseitenfläche der Membran 600 fluiddicht verbunden. Beide Fluidkammern 607a, 607b sind gegenüber voneinander angeordnet. Anders ausgedrückt wird über den ersten Abschnitt des Schafts 601 das erste elektrische Fluid in die Fluidkammern 607a, 607b gespült und die Fluidkammern 607a, 607b leiten das elektrisch leitfähige Fluid ausschließlich zu deren korrespondierenden Teilflächen.

Die erste und zweite Teilfläche 600a, 600b entsprechen einem Bereich der Innenseitenfläche der Membran 600, der sich von dem ersten Ende 622 der Membran 600 zu dem zweiten Ende 623 der Membran 600 erstreckt. Dadurch werden nicht alle Perforationslinien der Membran 600 mit dem elektrisch leitfähigen Fluid gespült. Dies ist nochmals in der Schnittdarstellung links oben in Fig. 1a gezeigt. Zu erkennen ist, dass die beiden Fluidkammern 607a, 607b zu jeder Seite einer Hochachse Az angeordnet sind. Die Teilflächen (nicht markiert) der Membran 600, die die Hochachse Az kreuzen, sind frei von einem Kontakt mit den Fluidkammern 607a, 607b. Somit würden im Falle einer Ablationsanwendung nur die Gewebeabschnitte behandelt, die mit den beiden Teilflächen 600a, 600b der Membran 600 korrespondieren.

In einer alternativen Variante der Ausführungsform können die beiden Fluidkammern 607a, 607b je nach Einsatzzweck beliebig entlang der Innenseitenfläche der Membran 600 angeordnet sein. Beispielsweise können beide Fluidkammern 607a, 607b unmittelbar aneinandergrenzen. In der dargestellten Variante überstreifen die beiden Fluidkammern jeweils einen Winkelbereich von etwa 90° (siehe Schnittdarstellung) und nehmen einen Gesamtwinkelbereich von etwa 180°. Alternativ kann, je nach Zusammensetzung des zu abladierenden Gewebes jede Fluidkammer 607a, 607b einen beliebigen Winkelbereich einnehmen, sodass sich ein Gesamtwinkelbereich beider Fluidkammern 607a, 607b zwischen etwa 45° und 360° ergibt (erster Winkelbereich etwa 90°, zweiter Winkelbereich etwa 180°, Gesamtwinkelbereich etwa 270°).

Alternativ kann eine Fluidkammer mit einem ersten elektrisch leitfähigen Fluid gespült werden, während die zweite Fluidkammer mit einem zweiten elektrischen leitfähigen Fluid gespült wird.

Alternativ können ein oder zwei zusätzliche Fluidkammern in dem Innenraum angeordnet werden. Die erste und zweite Fluidkammer können mit dem ersten elektrisch leitfähigen Fluid gespült werden, während die beide zusätzliche Fluidkammern über den ersten Schaft mit einem zweiten elektrisch leitfähigen Fluid gespült werden.

Alternativ kann eine Fluidkammer zuerst mit einem ersten elektrisch leitfähigen Fluid gespült werden, und daraufhin mit einem zweiten elektrisch leitfähigen Fluid.

Das erste elektrisch leitfähige Fluid ist beispielhaft als vasoaktive Flüssigkeit ausgebildet, welche insbesondere die elektrische Leitfähigkeit erhöht, und die zweite elektrische Flüssigkeit ist beispielhaft als vasoaktive Flüssigkeit ausgebildet, welche insbesondere die elektrische Leitfähigkeit vermindert.

Figur 1b zeigt eine schematische Darstellung einer Variante einer Ausführungsform mit einer Membran und einer Fluidkammer 607. Die abgebildete Variante weist einen Schaft 601 auf, der wiederum ein proximales Ende 620 und ein distales Ende 621 aufweist. Auf jeweils einem Teil eines ersten Abschnitts des Schafts 601 ist eine erste Elektrode 603a und zweite Elektrode 603b angeordnet. Die erste Elektrode 603a und die zweite Elektrode 603b werden von einer Membran 600 fluiddicht umschlossen. Anders ausgedrückt sind ein erstes Ende 622 und ein zweites Ende 623 der Membran mit dem Schaft 601 fluiddicht verbunden. Umschließt die Membran 600 den ersten Abschnitt des Schafts 601, bildet sich ein erster Innenraum zwischen der Membran 600 und dem Schaft 601. Die Membran 600 weist zumindest eine erste einzelne Perforation 602a auf, die in der dargestellten Variante als eine Vielzahl von Perforationslinien ausgebildet ist. Eine der Vielzahl von Perforationslinien ist mit 610a und einer gestrichelten Linienführung markiert. Jede der Vielzahl von Perforationslinien ist entlang einer Längsachse der Membran 600 ausgerichtet. Jede der Perforationslinien 610a beginnt auf dem mittigen Umfang der Membran 600 und endet in der dargestellten Variante an dem zweiten Ende 623 der Membran 600. Neben der ersten und zweiten Elektrode 603a, 603b sind außerhalb des ersten Innenraums auf dem Schaft 601 zwei weitere Gegenelektroden 604a, 604b angeordnet. Das proximale Ende 620 des Schafts 601 ist dazu eingerichtet, ein erstes elektrisch leitfähiges Fluid aufzunehmen und zum distalen Ende 621 des Schafts 601 zu leiten.

Der Innenraum weist eine erste und zweite Fluidkammer 607a, 607b auf. Diese werden durch eine, insbesondere fluidundurchlässige, Trennmembran 607 in dem Innenraum gebildet. Die Trennmembran 607 in dem Innenraum teilt den Innenraum in zwei in etwa gleich große Fluidkammern 607a, 607b.

Über den ersten und/oder zweiten Teil des Abschnitts kann die entsprechende Fluidkammer 607a, 607b befüllt werden. Vorliegend kann entweder eine der beiden oder es können beide Fluidkammer 607a, 607b befüllt werden. In der dargestellten Variante wird exemplarisch die erste Fluidkammer 607a gespült.

Die erste Fluidkammer 607a ist mit dem ersten Teil des ersten Abschnitts des Schafts 601 fluiddicht verbunden, auf dem die erste Elektrode 603a angeordnet ist. Die Trennmembran 607 schließt fluiddicht mit dem Schaft und der Innenseite der Membran 600 ab. Anders ausgedrückt wird über den ersten Teil des Abschnitts des Schafts 601 ein erstes elektrisch leitfähiges Fluid in die erste Fluidkammer 607a gespült und die erste Fluidkammer 607a leitet das erste elektrisch leitfähige Fluid zur der ersten Teilfläche.

Anders ausgedrückt wird über den ersten Abschnitt des Schafts 601 das erste elektrisch leitfähige Fluid in die erste Fluidkammer 607a gespült und die Fluidkammer 607a leitet das elektrisch leitfähige Fluid ausschließlich zu der ersten Teilfläche.

Die erste Teilfläche entspricht einem vollständigen Bereich der Innenseitenfläche der Membran 600, die sich von dem zweiten Ende 623 der Membran 600 zu einem mittleren Umfang der Membran 600 erstreckt. Anders ausgedrückt überstreift die erste Fluidkammer 607a einen Winkelbereich um die Längsachse der Membran von 360°.

Alternativ kann die erste Teilfläche mit der Vielzahl an Perforationslinien korrespondieren. Alternativ kann die erste Teilfläche mit ausschließlich einer Perforationslinie der Vielzahl von Perforationslinien oder mit einer beliebigen (benachbarten oder übernächst benachbarten) Auswahl von Perforationslinien der Vielzahl von Perforationslinien korrespondieren. Alternativ kann eine zusätzliche Fluidkammer vorgesehen sein/werden, die das erste oder ein zweites elektrisch leitfähiges Fluid zu einer zweiten Teilfläche, insbesondere zu einer anderen Auswahl von Perforationslinien, oder einer einzelnen Perforationslinie leitet, die nicht Teil der ersten Teilfläche ist.

In einer Variante mit einer ersten und einer zusätzlichen Fluidkammer kann jede Fluidkammer einen beliebigen Winkelbereich um die Längsachse der Membran einnehmen, sodass sich ein Gesamtwinkelbereich beider Fluidkammern zwischen 45° und 360° ergibt (erster Winkelbereich 180°, zweiter Winkelbereich 180°, Gesamtwinkelbereich 360°).

Die zusätzliche Fluidkammer kann in einer Variante eine Ausführungsform über den ersten Schaft mit einem zweiten elektrisch leitfähigen Fluid gespült werden, während die erste Fluidkammer mit dem ersten elektrisch leitfähigen Fluid gespült wird. Das erste elektrisch leitfähige Fluid ist beispielhaft als vasoaktive Flüssigkeit ausgebildet und die zweite elektrische Flüssigkeit ist beispielhaft als leitfähigkeitsmindernde Flüssigkeit ausgebildet.

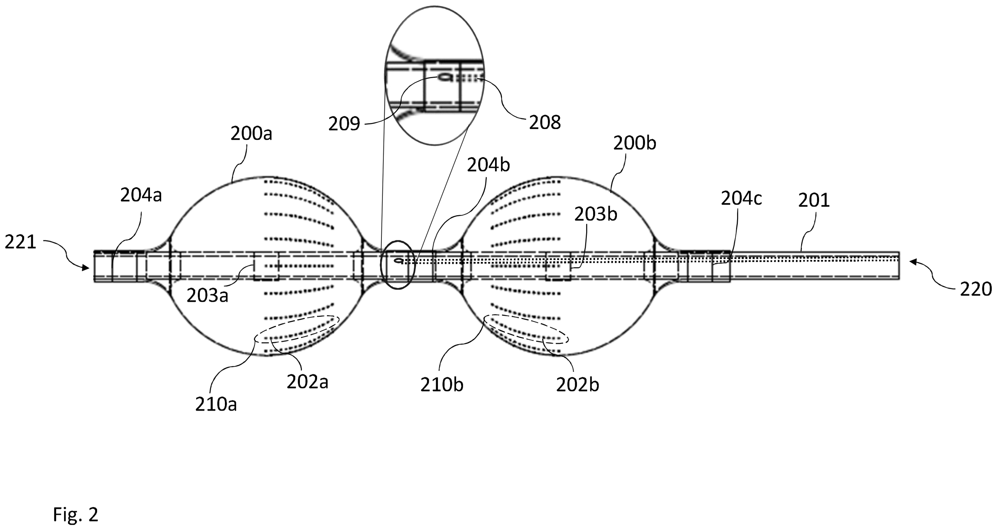

Figur 2 zeigt eine schematische Darstellung einer weiteren Variante einer Ausführungsform der Kathetervorrichtung mit zwei Membranen 200a, 200b. Die abgebildete Variante weist einen Schaft 201 auf, der wiederum ein proximales Ende 220 und ein distales Ende 221 aufweist. Auf einem ersten Abschnitt des Schafts 201 ist eine erste Elektrode 203a angeordnet. Der erste Abschnitt wird von einer Membran 200a fluiddicht umschlossen. Anders ausgedrückt sind ein erstes Ende und ein zweites Ende der Membran 200a mit dem Schaft 201 fluiddicht verbunden. Umschließt die Membran 200a den ersten Abschnitt des Schafts 201, bildet sich ein erster Innenraum zwischen der Membran 200a und dem Schaft 201. Die Membran 200a weist zumindest eine einzelne Perforation 202a auf, die in der dargestellten Variante als eine Vielzahl von Perforationslinien ausgebildet ist. Eine der Vielzahl von Perforationslinien ist mit 210a und einer gestrichelten Linienführung markiert. Jeder der Vielzahl von Perforationslinien ist entlang einer Längsachse der Membran 200a ausgerichtet. Jede der Perforationslinien beginnt auf dem mittigen Umfang der Membran 200a und endet in der dargestellten Variante an dem ersten Ende der Membran 200a.

Auf einem zweiten Abschnitt des Schafts 201 ist eine zweite Elektrode 203b angeordnet. Der zweite Abschnitt wird von einer Membran 200b fluiddicht umschlossen. Anders ausgedrückt sind ein erstes Ende und ein zweites Ende der Membran 200b mit dem Schaft 201 fluiddicht verbunden. Umschließt die Membran 200b den zweiten Abschnitt des Schafts 201 bildet sich ein zweiter Innenraum zwischen der Membran 200b und dem Schaft 201. Die Membran 200b weist zumindest eine einzelne Perforation 202b auf, die in der dargestellten Variante als eine Vielzahl von Perforationslinien ausgebildet ist. Eine der Vielzahl von Perforationslinien ist mit 210b und einer gestrichelten Linienführung markiert. Jede der Vielzahl von Perforationslinien ist entlang einer Längsachse der Membran 200b ausgerichtet. Jede der Perforationslinien beginnt auf dem mittigen Umfang der Membran 200b und endet in der dargestellten Variante an dem zweiten Ende der Membran 200b.

Neben der ersten Elektrode 203a und der zweiten Elektrode 203b sind, außerhalb des ersten und zweiten Innenraums, auf dem Schaft 201 drei Gegenelektroden 204a, 204b, 204c angeordnet. Das proximale Ende 220 des Schafts 201 ist dazu eingerichtet, ein elektrisch leitfähiges Fluid aufzunehmen und zum distalen Ende 221 des Schafts 201 zu leiten. Der Schaft 201 weist eine Wandung auf. Die Wandung weist im ersten Abschnitt des Schafts 201 eine erste Befüllungsöffnung (nicht dargestellt) auf, über die das elektrische leitfähige Fluid in den ersten Innenraum geleitet wird. Der erste Innenraum wird mit dem elektrisch leitfähigen Fluid gefüllt. Die Wandung weist im zweiten Abschnitt des Schafts 201 eine zweite Befüllungsöffnung (nicht dargestellt) auf, über die das elektrische leitfähige Fluid in den zweiten Innenraum geleiten wird. Der zweite Innenraum wird mit dem elektrisch leitfähigen Fluid gefüllt. Das elektrisch leitfähige Fluid tritt aus der Vielzahl von Perforationslinien jeder Membran 200a, 200b hinaus.

In der dargestellten Variante bildet sich, wenn die Vorrichtung in ein Lumen (nicht dargestellt) geführt wird, ein Zwischenraum zwischen der ersten und zweiten Membran 200a, 200b und der an der Vorrichtung grenzenden Gewebewand (nicht dargestellt). Tritt das elektrisch leitfähige Fluid aus der Vielzahl an Perforationslinien 202a, 202b aus, füllt sich der Zwischenraum mit dem elektrisch leitfähigen Fluid und steht in Kontakt mit der Gewebewand.

Wird ein elektrisches Signal zwischen der ersten Elektrode 203a und der Gegenelektrode 204b angelegt, erfolgt aufgrund des ausgetretenen elektrisch leitfähigen Fluids eine verbesserte Feldlinienführung in das zu abladierende Gewebe, welche in der Figur 6 näher erläutert wird.

Um die Belastung für das Lumen zu reduzieren, ist in der dargestellten Variante eine Ablauföffnung oder Drainageöffnung 209 auf dem Schaft 201 vorgesehen. Das elektrisch leitfähige Fluid wird aus dem Zwischenraum über die Ablauföffnung 209 in das Innere des Schafts 201 geleitet. In dem Schaft ist eine Ablaufleitung 208 vorgesehen, über die das in dem Zwischenraum befindliche elektrisch leitfähige Fluid zu dem proximalen Ende 220 des Schafts 201 geleitet wird.

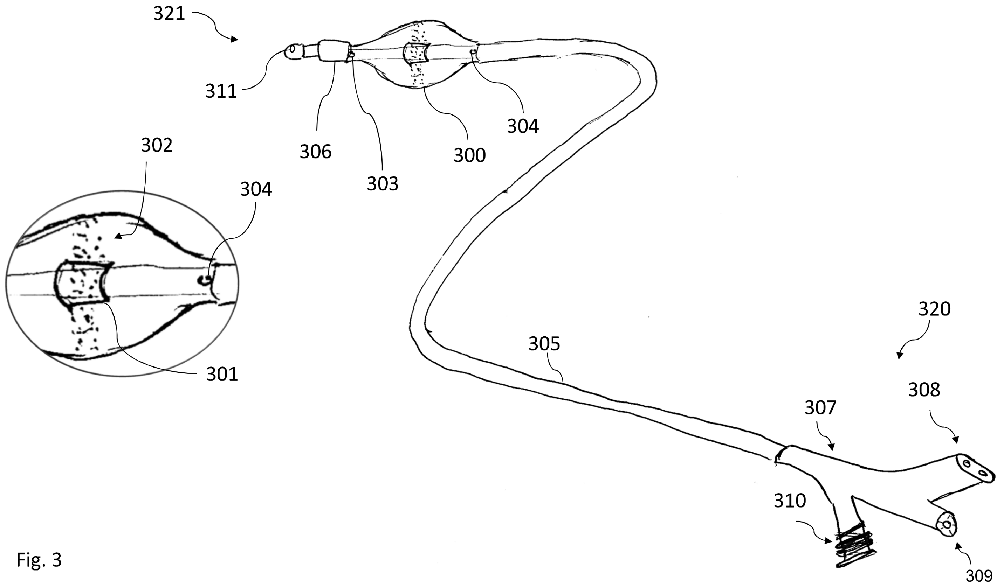

Figur 3 zeigt eine schematische Darstellung einer Variante einer Ausführungsform mit Bedieneinheit 307. Die abgebildete Variante weist einen Schaft 305 auf, der wiederum ein proximales Ende 320 und ein distales Ende 321 aufweist. Auf einem ersten Abschnitt des Schafts 305 ist eine erste Elektrode 301 angeordnet. Der erste Abschnitt wird von einer Membran 300 fluiddicht umschlossen. Anders ausgedrückt sind ein erstes Ende und ein zweites Ende der Membran 300 mit dem Schaft 305 fluiddicht verbunden. Umschließt die Membran 300 den ersten Abschnitt des Schafts 305 bildet sich ein erster Innenraum zwischen der Membran 300 und dem Schaft 305. Die Membran 300 weist zumindest eine einzelne Perforation auf, die in der dargestellten Variante als eine Vielzahl von Perforationen 302 ausgebildet ist. Die Vielzahl von Perforationen 302 ist entlang eines mittleren Umfangs der Membran 300 verteilt. Neben der ersten Elektroden 301 ist außerhalb des ersten Innenraums auf dem Schaft 305 eine Gegenelektrode 306 angeordnet.

Das proximale Ende 320 des Schafts 305 weist eine Bedieneinheit 307 auf sowie einen Zu- und Abfluss-Anschluss 310, über den in der dargestellten Variante ein elektrisch leitfähiges Fluid in den Schaft und zur Membran 300 geleitet wird.

Die Bedieneinheit 307 weist in der dargestellten Variante einen elektrischen Anschluss 308 auf, der mit einer in dem Schaft angeordneten elektrischen Leitung (nicht dargestellt) verbunden ist. Die elektrische Leitung ist mit der ersten Elektrode 301 und der einen Gegenelektrode 306 verbunden.

Die Bedieneinheit 307 weist eine als Hämostaseventil ausgebildete Einführungsöffnung 309 auf, über die ein Führungsdraht in den Schaft 305 und bis zu dem distalen Ende 321 geführt werden kann. Das distale Ende 321 weist eine Austrittöffnung 311 auf, aus der der Führungsdraht aus dem distalen Ende 321 geleitet werden kann.

Auf dem ersten Abschnitt des Schafts 305 ist neben der ersten Elektrode zumindest eine Befüllungsöffnung 304 angeordnet, die mit dem Inneren des Schafts 305 verbunden ist und aus der das elektrisch leitfähige Fluid in den ersten Innenraum gelangt und jenen befüllt. Auf dem ersten Abschnitt des Schafts 305 ist neben der ersten Elektrode zumindest eine Entlüftungsöffnung 303 angeordnet, die mit dem Inneren des Schafts 305 verbunden ist und Luft aus dem ersten Innenraum leitet, wenn der erste Innenraum mit dem elektrisch leitfähigen Fluid befüllt wird.

Aus der Vielzahl von Perforation 302 tritt das elektrisch leitfähige Fluid aus.

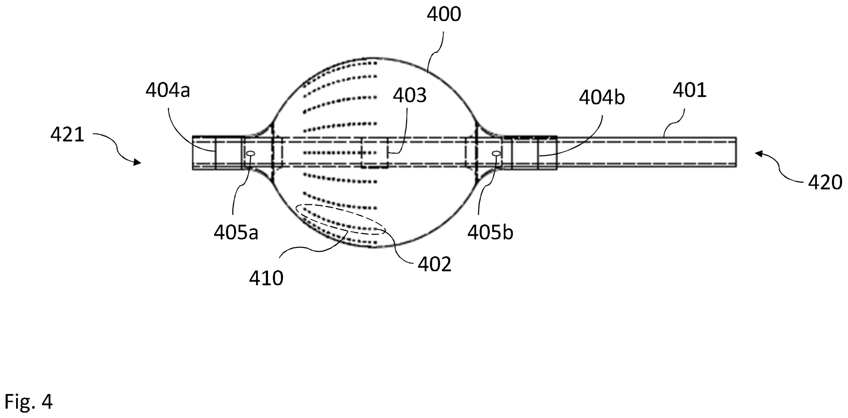

Figur 4 zeigt eine schematische Darstellung einer Variante einer Ausführungsform mit einer Entlüftungsöffnung 405a und einer Befüllungsöffnung 405b. Die abgebildete Variante weist einen Schaft 401 auf, der wiederum ein proximales Ende 420 und ein distales Ende 421 aufweist. Auf einem ersten Abschnitt des Schafts 401 ist eine erste Elektrode 403 angeordnet. Der erste Abschnitt wird von einer Membran 400 fluiddicht umschlossen. Anders ausgedrückt ist ein erstes Ende 122 und ein zweites Ende 123 der Membran mit dem Schaft fluiddicht verbunden. Umschließt die Membran 400 den ersten Abschnitt des Schafts 401, bildet sich ein erster Innenraum zwischen der Membran 400 und dem Schaft 401. Die Membran 400 weist zumindest eine einzelne Perforation 402 auf, die in der dargestellten Variante als eine Vielzahl von Perforationslinien ausgebildet ist. Eine der Vielzahl von Perforationslinien ist mit 410 und einer gestrichelten Linienführung markiert. Jede der Vielzahl von Perforationslinien ist entlang einer Längsachse der Membran 400 ausgerichtet. Jede der Perforationslinien beginnt auf dem mittigen Umfang der Membran 400 und endet in der dargestellten Variante an dem zweiten Ende der Membran 400.

Neben der ersten Elektroden 403 sind außerhalb des ersten Innenraums auf dem Schaft 401 zwei Gegenelektroden 404a, 404b angeordnet. Das proximale Ende 420 des Schafts 401 ist dazu eingerichtet, ein elektrisch leitfähiges Fluid, welches in der dargestellten Variante als elektrisch leitfähige Flüssigkeit ausgebildet ist, aufzunehmen und zum distalen Ende 421 des Schafts 401 zu leiten. Der erste Abschnitt des Schafts 401 ist dazu eingerichtet, über die Befüllungsöffnung 405b in der Wandung des Schafts 401 die elektrisch leitfähige Flüssigkeit in den ersten Innenraum zu leiten. Der erste Innenraum wird mit der elektrisch leitfähigen Flüssigkeit gefüllt und die erste Elektrode 403 umspült. Das elektrisch leitfähige Fluid tritt aus der Vielzahl von Perforationslinien hinaus. Wird ein elektrisches Signal zwischen der ersten Elektrode 403 und der Gegenelektrode 404a angelegt, erfolgt aufgrund der ausgetretenen elektrischen leitfähigen Flüssigkeit eine verbesserte Feldlinienführung in das zu abladierende Gewebe, welche in der Figur 6 näher erläutert wird.

Auf dem ersten Abschnitt der Membran 400 ist jeweils eine Entlüftungsöffnung 405a und eine Befüllungsöffnung 405b angeordnet. Wird die Vorrichtung in einer senkrechten Position gehalten und die elektrisch leitfähige Flüssigkeit über die Befüllungsöffnung in den ersten Innenraum geleitet, kann über die Entlüftungsöffnung Luft entweichen - die Membran 400 wird entlüftet.

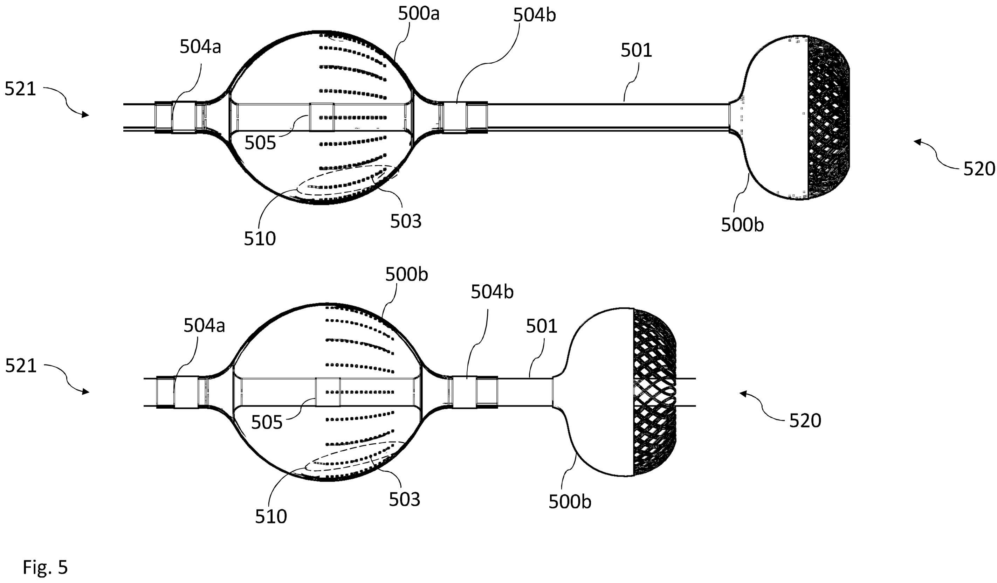

Figur 5 zeigt eine schematische Darstellung einer Variante einer Ausführungsform mit einem Begrenzungselement 500b.

Die abgebildete Variante weist einen Schaft 501 auf, der wiederum ein proximales Ende 520 und ein distales Ende 521 aufweist. Auf einem ersten Abschnitt des Schafts 501 ist eine erste Elektrode 505 angeordnet. Der erste Abschnitt wird von einer Membran 500a fluiddicht umschlossen. Anders ausgedrückt sind ein erstes Ende und ein zweites Ende der Membran 500a mit dem Schaft 501 fluiddicht verbunden. Umschließt die Membran 500a den ersten Abschnitt des Schafts 501, bildet sich ein erster Innenraum zwischen der Membran 500a und dem Schaft 501. Die Membran 500a weist zumindest eine einzelne Perforation 503 auf, die in der dargestellten Variante als eine Vielzahl von Perforationslinien ausgebildet ist. Eine der Vielzahl von Perforationslinien ist mit 510 und einer gestrichelten Linienführung markiert. Jede der Vielzahl von Perforationslinien ist entlang einer Längsachse der Membran 500a ausgerichtet. Jede der Perforationslinien beginnt auf dem mittigen Umfang der Membran 500a und endet in der dargestellten Variante an dem ersten Ende der Membran 500a.

Neben der ersten Elektroden 505 sind, außerhalb des ersten Innenraums, auf dem Schaft 501 zwei Gegenelektroden 504a, 504b angeordnet. Das proximale Ende 520 des Schafts 501 ist dazu eingerichtet, ein elektrisch leitfähiges Fluid aufzunehmen und zum distalen Ende 521 des Schafts 501 zu leiten. Der erste Abschnitt des Schafts 501 ist dazu eingerichtet, das elektrische leitfähige Fluid in den ersten Innenraum zu leiten. Der erste Innenraum wird mit dem elektrisch leitfähigen Fluid gefüllt. Das elektrisch leitfähige Fluid tritt aus der Vielzahl von Perforationslinien hinaus.

Auf einem zweiten Abschnitt des Schafts 501 ist eine Begrenzungsstruktur 500b angeordnet.

In der dargestellten Variante bildet sich, wenn die Vorrichtung in ein Lumen (nicht dargestellt) geführt wird, ein Zwischenraum zwischen der ersten Membran 500a, der Begrenzungsstruktur 500b und der an der Vorrichtung grenzenden Gewebewand (nicht dargestellt). Tritt das elektrisch leitfähige Fluid aus der Vielzahl an Perforationslinien aus, füllt sich der Zwischenraum mit dem elektrisch leitfähigen Fluid und steht in Kontakt mit der Gewebewand. Die Begrenzungsstruktur kann entlang einer Schaftachse verschoben werden, womit eingestellt werden kann, welcher Gewebeoberflächenanteil des Lumens mit der elektrisch leitfähigen Flüssigkeit in Kontakt steht.

Die Begrenzungsstruktur hemmt das aus der Vielzahl von Perforationslinien ausgetretene elektrisch leitfähige Fluid an der Ausbreitung entlang einer Lumenachse, wenn der Zwischenraum mit dem Fluid gefüllt wird.

Wird ein elektrisches Signal zwischen der ersten Elektrode 505 und der Gegenelektrode 504b angelegt, erfolgt aufgrund des ausgetretenen elektrisch leitfähigen Fluids eine verbesserte Feldlinienführung in das zu abladierende Gewebe, welche in der Figur 6 näher erläutert wird.

Figur 6 zeigt eine schematische Darstellung einer Variante einer Ausführungsform mit einem Feldlinienverlauf 511 während der Ablation. In der dargestellten Anwendung wurde die Vorrichtung aus der Figur 5 in ein Gewebelumen G eingeführt. Die Vorrichtung ist mit einer (nicht dargestellten) Signalgeneratoranordnung verbunden. Die Signalgeneratoranordnung ist mit einer (nicht dargestellten) Steuer- und Auswerteeinheit verbunden. Eine Fluidzufuhreinheit (nicht dargestellt) ist mit der Vorrichtung und der Steuer- und Auswerteeinheit verbunden und dazu eingerichtet, ein elektrisches leitfähiges Fluid bereitzustellen.

Die Steuer- und Auswerteinheit ist dazu eingerichtet, einen Volumenstrom der Fluidzufuhreinheit, insbesondere nach einer Einstellung and der Steuer- und Auswerteeinheit durch einen Nutzer, vorzugeben. Daraufhin füllt sich der erste Innenraum mit dem elektrisch leitfähigen Fluid und es tritt aus der Vielzahl von Perforationslinien aus. Der Zwischenraum, der mit dem elektrisch leitfähigen Fluid befüllt ist, ist durch die Schraffur (diagonal-gepunkte Linien einer Lumenwand zur gegenüberliegenden Lumenwand) in der Figur 6 dargestellt. Wird nun ein elektrisches Signal zwischen die ersten Elektrode 505 und der Gegenelektrode 504b angelegt, ergibt sich eine Feldlinien-Verteilung, von der exemplarisch eine Feldlinie 511 dargestellt ist. Aufgrund des direkten Kontakts des elektrisch leitfähigen Fluids mit dem Gewebe G und des Signalpfadzusammenschluss über das elektrisch leitfähige Fluid wird eine tieferes Eindringen der Feldlinien in das Gewebe erreicht, bei gleichzeitiger Kühlung des zu abladierenden Gewebes.

Alternativ kann der Zwischenraum zuerst mit einem flüssigen Wirkstoff wie Histamin gespült werden. Das Histamin wirkt im Gewebe und erhöht dessen elektrische Leitfähigkeit. Daraufhin kann von einem Nutzer an der Steuer- und Auswerteeinheit vorgegeben werden, dass eine zweite elektrisch leitfähige Flüssigkeit von der Fluidzufuhreinheit bereitzustellen ist oder bereitgestellt werden soll. Die zweite elektrisch leitfähige Flüssigkeit verdrängt den flüssigen Wirkstoff aus dem Innenraum der Membran 500 und aus dem Zwischenraum.

Wird nun ein elektrisches Signal zwischen die ersten Elektrode 505 und der Gegenelektrode 504b angelegt, ergibt sich eine andere Feldlinienverteilung (nicht dargestellt). Aufgrund des direkten Kontakts des zweiten elektrisch leitfähigen Fluids mit dem Gewebe G und des Signalpfadzusammenschlusses über das elektrisch leitfähige Fluid sowie der zuvor durch den flüssigen Wirkstoff erhöhten elektrischen Leitfähigkeit des Gewebes, wird eine tieferes Eindringen der Feldlinien in das Gewebe erreicht als ohne vorherige Applikation eines Wirkstoffs, bei gleichzeitiger Kühlung des zu abladierenden Gewebes.

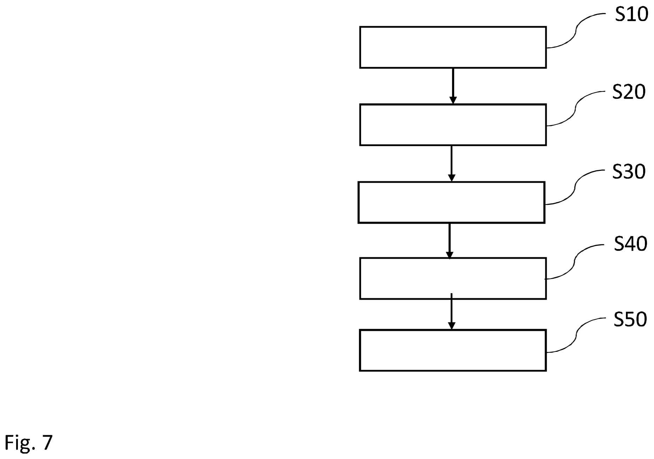

Fig. 7 zeigt ein Verfahren zur Abgabe elektrischer Energie an Gewebe. Das Verfahren umfasst ein Bereitstellen (Schritt S10) eines Systems. Das System weist eine Applikationsvorrichtung auf, die dazu eingerichtet ist, ein zu empfangendes elektrisches Signal und eine zu empfangende vasoaktive, insbesondere flüssige, Substanz, insbesondere Histamin, an/in das Gewebe weiterzuleiten. Das System weist eine mit der Applikationsvorrichtung verbundene oder verbindbare Signalgeneratoranordnung auf. Das System weist eine mit der Applikationsvorrichtung und/oder Signalgeneratoranordnung verbundene oder verbindbare Steuer- und Auswerteeinheit auf. Das System weist eine mit der Applikationsvorrichtung und/oder Steuer- und Auswerteeinheit verbundene oder verbindbare Fluidzufuhreinheit auf, die dazu eingerichtet ist, die vasoaktive Substanz bereitzustellen. Das Verfahren umfasst ein Kontaktieren (Schritt S20) der Applikationsvorrichtung mit dem Gewebe. Das Verfahren umfasst ein Einstellen (Schritt S30) der Steuer- und Auswerteeinheit zur Einstellung des Volumenstroms. Das Verfahren umfasst ein Applizieren (Schritt S40) der vasoaktiven Substanz. Das Verfahren umfasst ein Ansteuern (Schritt S50) der Steuer- und Auswerteeinheit zur Erzeugung eines elektrischen Signals.

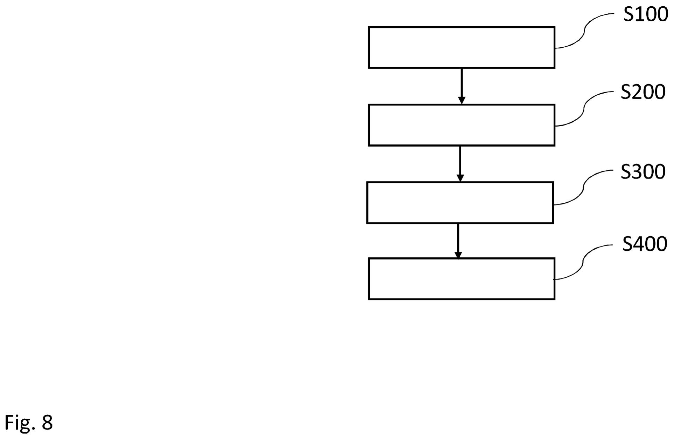

Fig. 8 zeigt ein Verfahren zur irreversiblen Ablation, umfassend die Schritte: Bereitstellen (Schritt S100) eines Systems aufweisend: eine Vorrichtung wie sie hierin beschrieben wurde; eine mit der Vorrichtung verbundene oder verbindbare Signalgeneratoranordnung, eine mit der Vorrichtung und/oder Signalgeneratoranordnung verbundene oder verbindbare Steuer- und Auswerteeinheit, und eine mit der Vorrichtung und/oder Steuer- und Auswerteeinheit verbundenen oder verbindbaren Fluidzufuhreinheit, dazu eingerichtet ein elektrisches leitfähiges Fluid bereitzustellen; Einführen (Schritt S200) der Vorrichtung in ein Lumen eines Patienten bis zu einem zu abladierenden Gewebeabschnitt; Einstellen (Schritt S300) eines Volumenstroms an der Fluidzufuhreinheit; Ansteuern (Schritt S400) der Steuer- und Auswerteeinheit zur Erzeugung eines elektrischen Signals.

## Patentansprüche

1. Eine Applikationsvorrichtung, insbesondere Kathethervorrichtung, beispielsweise zur Verwendung in der Chirurgie, aufweisend:
- einen Schaft (101; 201), dazu eingerichtet, zumindest ein elektrisch leitfähiges Fluid aufzunehmen,
- zumindest eine auf einem ersten Abschnitt des Schafts angeordnete erste Elektrode (103; 203a),
- zumindest eine erste Gegenelektrode (104b; 204b),
- eine erste elektrische Leitung, die mit der zumindest einen ersten Elektrode (103; 203a) und der zumindest einen ersten Gegenelektrode (104b; 204b) verbunden ist, dazu eingerichtet, ein zu empfangendes elektrisches Signal an die zumindest eine erste Elektrode (103; 203a) und die zumindest eine erste Gegenelektrode (104b; 204b) weiterzuleiten,
- zumindest eine erste Membran (100; 200a), dazu eingerichtet,
- den ersten Abschnitt des Schafts zu umschließen,
- mit dem Schaft (101; 201) fluiddicht abzuschließen, und
- zwischen der zumindest einen ersten Membran (100; 200a) und dem ersten Abschnitt des Schafts einen ersten Innenraum zu bilden,
wobei der erste Abschnitt des Schafts dazu eingerichtet ist, das zumindest eine elektrisch leitfähige Fluid in den Innenraum zu leiten, wobei eine erste Wandung der zumindest einen ersten Membran (100) dazu eingerichtet ist, zumindest teilweise fluiddurchlässig zu sein.

2. Die Vorrichtung nach Anspruch 1, wobei
die zumindest eine erste Membran, insbesondere die erste Wandung, zumindest eine erste Perforation (102; 202a) aufweist und das elektrisch leitfähige Fluid aus der zumindest einen ersten Perforation (102; 202a) austreten kann, und/oder
die Vorrichtung ferner aufweist:
- zumindest eine auf einem zweiten Abschnitt des Schafts angeordnete und mit der ersten elektrischen Leitung verbundene zweite Elektrode (203b), und/oder
- zumindest eine zweite, insbesondere zumindest eine zweite Perforation (210b) aufweisende, Membran (200b), dazu eingerichtet,
- den zweiten Abschnitt des Schafts (101; 201) zu umschließen,
- mit dem Schaft (101; 201) fluiddicht abzuschließen, und
- zwischen der zumindest einen zweiten Membran (200b) und dem zweiten Abschnitt des Schafts einen zweiten Innenraum zu bilden, und/oder
wobei der zweite Abschnitt des Schafts dazu eingerichtet ist, das elektrisch leitfähige Fluid in den zweiten Innenraum zu leiten, wobei eine zweite Wandung der zumindest einen zweiten Membran dazu eingerichtet ist, fluiddurchlässig zu sein, und/oder
- wobei das elektrisch leitfähige Fluid aus der zumindest einen zweiten Perforation (202b) der zumindest einen zweiten Membran austritt oder austreten kann, und/oder
- wobei der Schaft (101; 201) ein distales Ende und ein proximales Ende aufweist und die zumindest eine erste Membran (100; 200a) zwischen dem distalen Ende (121; 221) und dem proximalen Ende (120; 220) angeordnet ist, und/oder die zumindest eine zweite Membran (200b) zwischen der zumindest einen ersten Membran (200a) und dem proximalen Ende (220) angeordnet ist.

3. Die Vorrichtung nach Anspruch 1 oder 2, wobei die Vorrichtung ferner aufweist:
- zumindest eine Begrenzungsstruktur (500b), die einen zweiten Teil des Schafts fluiddicht umschließt, dazu eingerichtet, das aus der ersten Wandung, insbesondere das aus der zumindest einen ersten Perforation, austretende elektrische leitfähige Fluid in einer Ausbreitungsrichtung zu hemmen oder zu begrenzen und/oder entlang des Schafts verschiebbar zu sein oder verschoben zu werden, wobei die zumindest eine Begrenzungsstruktur (500b) zwischen der zumindest einen ersten Membran (503) und dem proximalen Ende (520) angeordnet ist.

4. Die Vorrichtung nach einem der vorherigen Ansprüche, aufweisend:
eine Ablauföffnung (209), die auf dem Schaft (201) zwischen der zumindest einen ersten Membran (500a) und der zumindest einen Begrenzungsstruktur (500b) oder zwischen der zumindest einen ersten Membran (200a) und der zumindest einen zweiten Membran (220b) angeordnet ist, dazu eingerichtet, das aus der ersten Wandung, insbesondere aus der zumindest einen ersten Perforation (202a), und/oder aus der zweiten Wandung, insbesondere aus der zumindest einen zweiten Perforation (202b), ausgetretene elektrisch leitfähige Fluid abzuführen, wobei der Schaft (201) dazu eingerichtet ist, das abgeführte elektrisch leitfähige Fluid, insbesondere zu/über dem/das proximale Ende (220), aus dem Schaft (201) zu leiten; und/oder
- wobei zumindest eine erste und/oder zumindest eine zweite und/oder zumindest eine dritte Gegenelektrode zwischen: dem distalen Ende und der zumindest einen ersten Membran, oder zwischen der zumindest einen ersten Membran und dem proximalen Ende, oder zwischen der zumindest einen ersten und der zumindest einen zweiten Membran, oder zwischen der zumindest einen zweiten Membran und dem proximalen Ende, oder zwischen der zumindest einen ersten Membran und der zumindest einen Begrenzungsstruktur angeordnet ist; und/oder
- die zumindest eine erste und/oder zweite und/oder dritte Gegenelektrode als Körperelektrode ausgebildet ist.

5. Die Vorrichtung nach einem der vorherigen Ansprüche, wobei die zumindest eine erste (102; 202a) und/oder die zumindest eine zweite (202b) Perforation ausgebildet ist als:
- zumindest eine Perforationslinie (110; 210a), die entlang einer Längsachse der Membran (100; 200a; 200b), insbesondere von einem mittleren/mittigen Membranumfang beginnend, in Längsachsrichtung, hin zu einem ersten oder zweiten Ende der Membran, ausgerichtet ist; und/oder
- eine Vielzahl von Perforationslinien, die jeweils entlang einer Längsachse der Membran, insbesondere von einem mittleren/mittigen Umfang beginnend, in Längsachsrichtung, hin zu einem ersten oder zweiten Ende der Membran, ausgerichtet sind; und/oder
- eine Vielzahl von Perforationen (302), die entlang oder quer eines, insbesondere mittleren/mittigen, Umfangs der Membran angeordnet oder verteilt sind.

6. Die Vorrichtung nach einem der vorherigen Ansprüche, wobei der erste und/oder der zweite Abschnitt eine Befüllungsöffnung (304) aufweist/aufweisen, wobei über das proximale Ende der erste und/oder der zweite Innenraum mit dem elektrisch leitfähigen Fluid befüllt wird; und/oder
wobei der erste und/oder der zweite Abschnitt eine Entlüftungsöffnung (405a) aufweist/aufweisen, und/oder der Schaft eine in dem Schaft angeordnete und mit der Entlüftungsöffnung verbundene Entlüftungsleitung aufweist.

7. Die Vorrichtung nach einem der vorherigen Ansprüche, wobei die Vorrichtung, der erste Abschnitt und/oder der zweite Abschnitt und/oder eine Außenseite der ersten und/oder zweiten Membran einen Fluidruckmess-Sensor und/oder einen Fluidflussmess-Sensor aufweist, jeweils dazu eingerichtet, Mess-Daten bereitzustellen, wobei die Mess-Daten über eine in dem Schaft vorgesehene oder angeordnete zweite elektrische Leitung ausgelesen werden können.

8. Die Vorrichtung nach einem der vorherigen Ansprüche, wobei das proximale Ende (320) des Schafts (305) eine Bedieneinheit (307) aufweist, wiederum aufweisend:
- einen Zu- und Abfluss-Anschluss (310), dazu eingerichtet, ein zu empfangendes elektrisch leitfähiges Fluid in den Schaft zu leiten, insbesondere den/die Innenraum/Innenräume mit einem/dem zu empfangenden elektrisch leitfähigen Fluid zu befüllen, und ausgetretenes und abgeführtes elektrisch leitfähiges Fluid aus dem Schaft zu leiten; und/oder
- einen mit der ersten und/oder der zweiten elektrischen Leitung verbundenen elektrischen Anschluss (308), dazu eingerichtet, zu empfangende elektrische Signale an die erste elektrische Leitung zu senden und/oder elektrische Signale, insbesondere Mess-Daten von dem Fluiddruckmess-Sensor und/oder Fluidflussmess-Sensor, mittels der zweiten elektrischen Leitung zu empfangen und weiterzusenden; und/oder
- eine, insbesondere als Hämostaseventil ausgebildete, Einführungsöffnung (309), dazu eingerichtet, einen Führungsdraht aufzunehmen.

9. Die Vorrichtung nach einem der vorherigen Ansprüche, wobei der erste und/oder zweite Innenraum zumindest eine erste Fluidkammer aufweist, die dazu eingerichtet ist, den ersten und/oder zweiten Abschnitt des Schafts fluiddicht zu umschließen, und das elektrisch leitfähige Fluid zu zumindest einer Teilfläche einer Innenseitenfläche der ersten und/oder zweiten Membran zu leiten.

10. Die Vorrichtung nach einem der vorherigen Ansprüche, wobei die zumindest eine erste Elektrode, die zumindest eine zweite Elektrode, die zumindest eine erste, zweite, dritte Gegenelektrode, die zumindest eine erste Membran und/oder die die zumindest eine zweite Membran, insbesondere die zumindest eine erste und/oder zweite Perforation, ein bioresorbierbares, insbesondere in Köperflüssigkeiten lösliches, Material aufweist oder mit einem bioresorbierbaren, insbesondere einem in Köperflüssigkeiten löslichen, Material oder einer entsprechenden Materialschicht bedeckt ist.

11. Ein System zur Ablation eines Gewebes, aufweisend:
- mindestens eine Vorrichtung nach einem der Ansprüche 1 bis 10,
- mindestens eine mit der Vorrichtung verbundene oder verbindbare Signalgeneratoranordnung,
- mindestens eine mit der Vorrichtung und/oder Signalgeneratoranordnung verbundene oder verbindbare Steuer- und Auswerteeinheit, und
- mindestens eine mit der Vorrichtung und/oder Steuer- und Auswerteeinheit verbundene oder verbindbare Fluidzufuhreinheit, dazu eingerichtet, ein elektrisch leitfähiges Fluid bereitzustellen.

12. Das System nach Anspruch 11, wobei die Steuer- und Auswerteinheit dazu eingerichtet ist, einen Volumenstrom der Fluidzufuhreinheit vorzugeben, wobei die Fluidzufuhreinheit dazu eingerichtet ist, das elektrisch leitfähige Fluid entsprechend der Vorgabe bereitzustellen und mittels des Fluiddruckmess-Sensors und/oder des Fluidfluss-Sensors einen Fluiddruck und/oder Fluidfluss zu messen.

13. Das System nach Anspruch 12, wobei die Steuer- und Auswerteeinheit dazu eingerichtet ist, aus dem gemessenen Fluiddruck und/oder Fluidfluss ein Maß für die Formschlüssigkeit zwischen der Membran, insbesondere der zumindest einen Perforation, und dem zu abladierenden Gewebe zu bestimmen und auszugeben.

14. Das System nach einem der Ansprüche 11 bis 13, wobei das, insbesondere ein erstes und/oder zweites, elektrisch leitfähige Fluid als elektrisch leitfähige Flüssigkeit und/oder als elektrisch leitfähiger, flüssiger Wirkstoff ausgebildet ist, insbesondere als Histamin, die bzw. der bei Kontakt mit einem Gewebe dessen elektrische Leitfähigkeit verändert, insbesondere erhöht oder vermindert; und/oder wobei die Signalgeneratoranordnung einen ersten Signalgenerator zur Erzeugung eines Radiofrequenz, RF, -Signals und einen zweiten Signalgenerator zur Erzeugung eines Signals für eine gepulste Feldablation aufweist.

15. Das System nach Anspruch 14, wobei die Steuer- und Auswerteeinheit dazu eingerichtet ist, den ersten Signalgenerator und/oder den zweiten Signalgenerator zur Abgabe eines Signals anzusteuern.
